# EUROPEAN PATENT SPECIFICATION

(11) **EP 1 523 955 B1**
(45) Date of publication and mention of the grant of the patent: **14.12.2011**
(21) Application number: 03023620.2
(22) Date of filing: 16.10.2003
(51) Int. Cl.: A61C 8/00, A61F 2/00, A61B 19/02

(54) **Package for preserving a medical device, in particular a dental implant**
Verpackung zur Konservierung einer medizinischen Vorrichtung, insbesondere eines Zahnimplantates
Emballage pour la conservation d'un dispositif médical, en particulier un implant dentaire

(43) Date of publication of application: 20.04.2005
(73) Proprietor: Straumann Holding AG, 4437 Waldenburg (CH)
(72) Inventor: Jemelin, Vincent, 4312 Magden Ag (CH); Kunz, Armin, 8200 Schaffhausen (CH); Keller, Olivier, 8604 Hegnau (CH); Baumann, Reinold, 9430 St.Margrethen (CH)
(74) Representative: Modiano, Micaela Nadia

(56) References cited:
- EP-A- 0 681 795
- WO-A-03/059190
- US-A1- 2003 221 977
- US-B1- 6 261 097

## Description

### FIELD OF THE INVENTION

The present invention relates in general to a package for preserving a medical device or the like, and in particular to a package with an external capsule for preserving a dental implant, the external capsule being provided with a mechanism for releasing the dental implant such as to minimize the danger of contamination of the dental implant.

### BACKGROUND ART

A package with an external capsule for preserving a dental implant is known from US Patent No. 6 261 097 which was assigned to the assignee of the present invention.

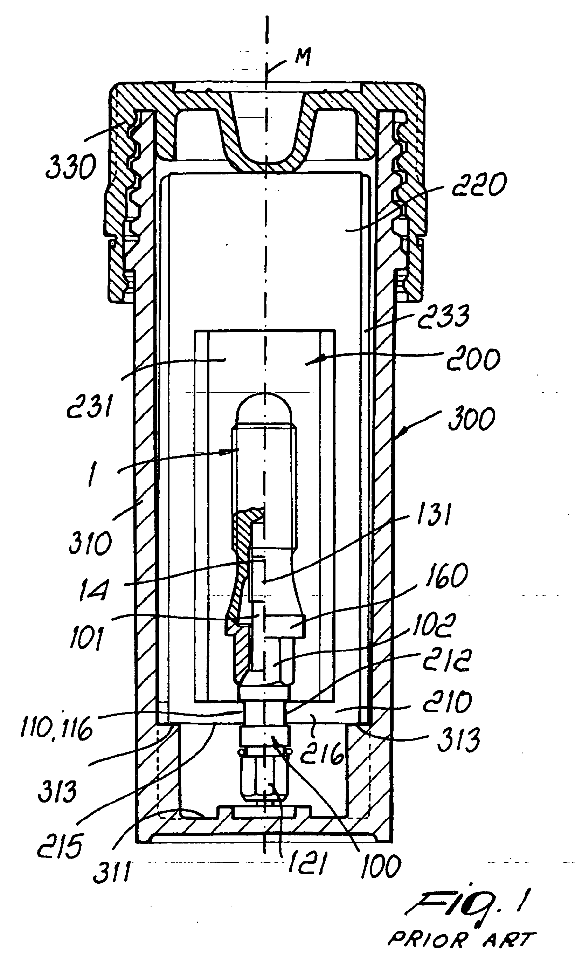

According to US Patent No. 6 261 097, which is reflected by the precharacterizing portion of claim 1, there is provided, as shown in prior art Fig. 1, an implant 1 and a holding element 100 with an extension element 121 releasably associated with the implant 1. In the assembled state, an ampoule 200, with the implant 1 held therein by the holding element 100, is inserted into an external capsule 300. The external capsule 300 comprises a hollow cylinder 310, the base 311 of which is closed, and a screw-on closure cap 320. On the inside of the cylinder 310, parallel to and at a distance from the base 311, there is a support shoulder 313, which is intended to act as an axial stop for the first planar base side on a fixing part 210 of the inserted ampoule 200. In this case, the support shoulder 313 comprises four webs which are offset through 90 DEG in each case. The closure cap 320 points towards a stand part 220 of the ampoule 200. At most in the region of the clearance between the second planar base side on the stand part 220 and the closure cap 320, the ampoule 200 can move on the axis M and otherwise lies in a stable position in the external capsule 300 in the event of vibrations.

The implant 1 is held by a holding element 100 with a screw 101 and a sleeve part 102. An externally threaded part 131 of the screw 101, which projects through the sleeve part 102, engages in an internally threaded bore 14 on the implant 1, while a mating shoulder 161 of a shoulder part 160 of the sleeve part 102 rests on an implant shoulder. A fixing part 110 of the holding element 100 is latched into the fixing part 210 of an ampoule 200, i. e. the cylindrical section 116 of the holding element 100 is clamped in a laterally open indent 212 in the ampoule 200 and is surrounded laterally by the two jaws 215, 216. The annular shoulders of the holding element 100 bear against the fixing part 210 on both sides. In this way, the implant 1 is held in line with the center axis M inside the ampoule 200 without coming into contact with the ampoule 200.

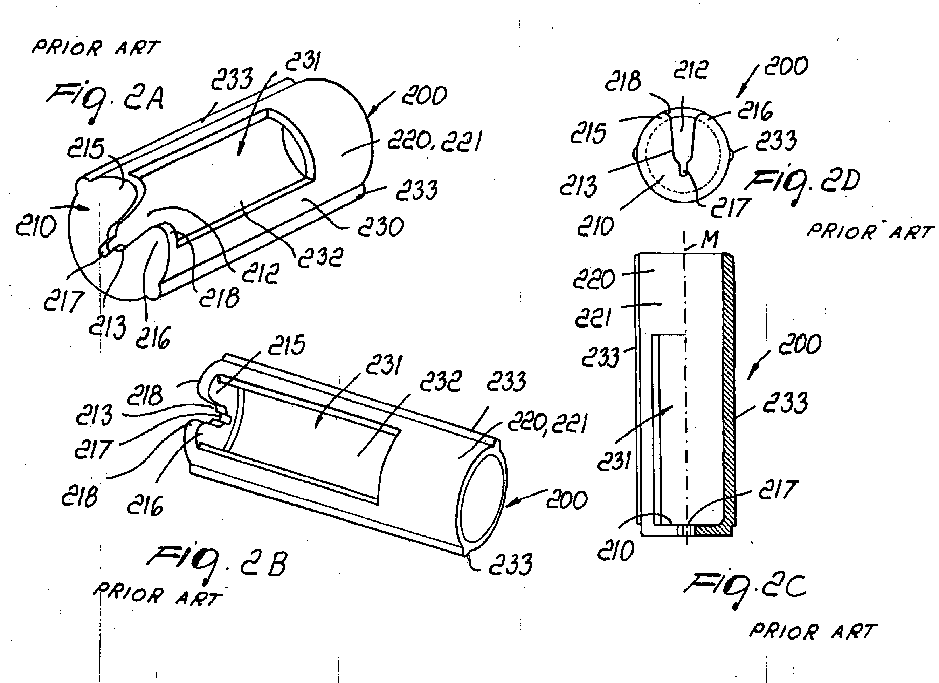

The ampoule 200 for accommodating the implant 1, which is shown in more details in prior art Figs. 2A through 2D and which is in principle cylindrical, has the fixing part 210 on the first planar base side and the stand part 220 on the opposite, second planar base side. A cylindrical casing 230 extends between the fixing part 210 and the stand part 220, in which casing there is a large-area cutout 231 which runs from the fixing part 210 as far as the stand part 220 and extends, for example, over a quarter to a half of the radial circumference of the ampoule 200. The implant 1, which is held in the ampoule 200, can be pulled out through this lateral cutout 231. Thus, the cylindrical casing 230 which remains in the region of the cutout 231, is in the form of an open shell 232, while in the stand part 220 the cylindrical casing 230 is entirely retained, where it produces, as it were, a tubular section 221. The second planar base side may be open or closed or partially open.

The cutout 231 extends as far as the fixing part 210, which is in the form of a circular end plate, so that the associated first planar base side is largely closed and the cylindrical casing 230 is perpendicular to the fixing part 210. The laterally open indent 212 is situated in the fixing part 210, and this indent 212, together with the cutout 231, face in the same direction. The indent 212 is in principle in the form of a slot with rounded sections 218 at the peripheral entry. In the region of the theoretical center axis M the indent 212 has a constriction 213, behind which the indent 212 widens in the manner of a semicircle. The result is that the two jaws 215, 216 are mutually opposite on the fixing part 210. Beyond the indent 212, cutting further into the fixing part 210 towards the cylindrical casing 230, there is an expansion groove 217, so that when an implant 1 or a holding element 100 bearing the implant 1 is being pressed in and out between the jaws 215, 216, the latter are better able to spread apart elastically. When the holding element 100 is being pressed in, after its cross-section has overcome the constriction 213, the holding element 100 latches into the indent 212 and the jaws 215, 216 move closer together again. Owing to the asymmetric distribution of material, the center of gravity of the ampoule 200 lies outside the center axis M, so that an ampoule 200 which is lying horizontally and hence rolling quickly comes to a halt. In order additionally to prevent the ampoule 200 from rolling off the surface, in each case one bead 233 is provided on the outside of the cylindrical casing 230, in an axial position and parallel to the edges of the cutout 231. The plane which extends between the two beads 233 divides the tubular ampoule 200 into two longitudinal halves. With the stand part 220 at the bottom, the ampoule 200 can be placed vertically resting on the second planar base side. A suitable material for the ampoule 200 is a biocompatible plastic.

Unfortunately, however, the known solution of US Patent No. 6 261 097, in particular in case of handling with a fluid, is sensitive to contamination prior to implanting the dental implant upon extracting the ampoule with the holding element and dental implant from the external capsule. Therefore, particular procedures must be implemented to make sure that the extraction step from the external capsule is performed in a sterile environment. In addition, the extraction process of the holding element from the external capsule is complicated.

Another capsule or package for a dental implant is known from WO-A-02 30315. The known package according to WO-A-02 30315 comprises a protective housing and a holder arranged to support the dental implant spaced from the lateral walls of the protective housing. The holder is arranged for sliding motion into and out of the protective housing, such that, when the holder is extracted from the housing it allows access to the dental implant. The latter solution is even more sensitive to contamination as the dental implant is fully unprotected upon extraction from the protective housing.

An assembly for securing a dental implant within a package is known from WO 03/059 190 A1.

### SUMMARY OF THE INVENTION

In view of the foregoing it is an object of the present invention to provide for a package for preserving a medical device, in particular a dental implant, which avoids the drawbacks of the prior art and which provides for safe and easy handling the medical device or the dental implant prior to implantation.

The above object as well as further objects which will become apparent hereinafter are achieved by a package for preserving a medical device and the like as defined in the appended claims.

### BRIEF DESCRIPTION OF THE DRAWINGS

The foregoing and other objects, features, and advantages of the invention, as well as presently preferred embodiments thereof, will become more apparent from a reading of the following description, in connection with the accompanying drawings in which:
Fig. 1 is a sectional view of a package with an external capsule for preserving a dental implant according to the prior art of US Patent No. 6 261 097;
Figs. 2A through 2D show various views of an ampoule as known from US Patent No. 6 261 097 for use with the external capsule according to Fig. 1;
Fig. 3 shows a partially cutaway view of a first embodiment of a package with an external capsule for preserving a medical device, in particular a dental implant, according to the present invention;
Figs. 4A through 4C show the first embodiment of the present invention in use from the closed state of the external capsule with an ampoule holding the dental implant up to the separation of the ampoule with the dental implant from the capsule;
Fig. 5 shows a sectional view of a second embodiment of a package with an external capsule for preserving a medical device, in particular a dental implant, according to the present invention;
Figs. 6A through 6C show the second embodiment of the present invention in use from the closed state of the external capsule with an ampoule holding the dental implant up to the separation of the ampoule with the dental implant from the capsule;
Fig. 7 shows a cross sectional view of a third embodiment of a package with an external capsule for preserving a medical device, in particular a dental implant, according to the present invention;
Figs. 8A through 8D show the third embodiment of the present invention in use from the closed state of the external capsule with an ampoule up to the separation of the ampoule from the capsule;
Fig. 9A shows a cross sectional view of a fourth embodiment of a package with an external capsule for preserving a medical device, in particular a dental implant, according to the present invention;
Fig. 9B shows a perspective view of the external capsule of the fourth embodiment with a bayonet lock for receiving the cap;
Fig. 9C shows a sectional view of the cap according to the fourth embodiment of the present invention; and
Figs. 10A through 10C show the fourth embodiment of the present invention in use from the closed state of the external capsule with an ampoule up to the separation of the ampoule from the capsule.

### DESCRIPTION OF THE PREFERRED EMBODIMENTS

Basically the embodiments disclosed hereinbelow may provide for a click and drop mechanism for separating the ampoule with the implant hold therein from a cap or a transport means when the ampoule/holder combination is removed with the cap or the transport means from an external capsule.

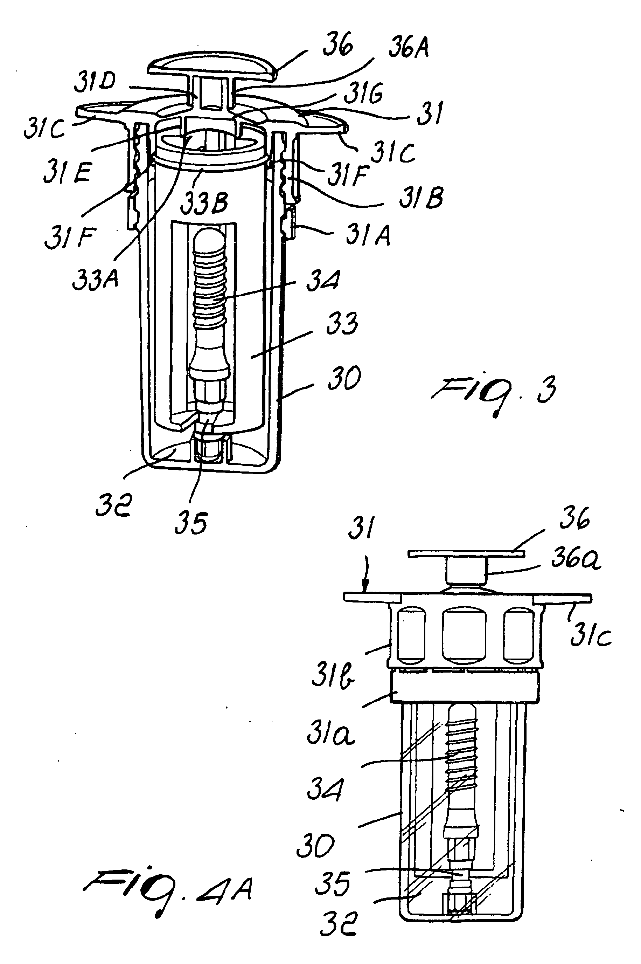

With reference to Figs. 3 and 4A through 4C there is shown a first embodiment of a package with an external capsule for preserving a medical device, in particular a dental implant, according to the present invention. As shown in Fig. 3, the external capsule, which may have a substantially cylindrical shape, is indicated by reference numeral 30 and is closed by a cap 31. The capsule 30 may be made of a transparent material, as shown in Fig. 4A or an opaque material. Transparent materials are preferred.

Preferably the cap 31 engages the open portion of the capsule 30 threadingly to provide for a sealed enclosure within the capsule 30. Advantageously the sealed enclosure may be filled with a fluid 32, such as an electrolyte or an aqueous solution, and houses an ampoule indicated with reference numeral 33 which holds a dental implant 34 via a holding element 35. The ampoule 33, the dental implant 34 and the holding element 35 can be devised and interact in the manner described in the prior art US Patent No 6 261 097 and explained hereinabove. The person skilled in the art will however appreciate that the ampoule 33, the dental implant 34 and the holding element 35 may have other alternative configurations without departing from the scope of the present invention as defined in the appended claims.

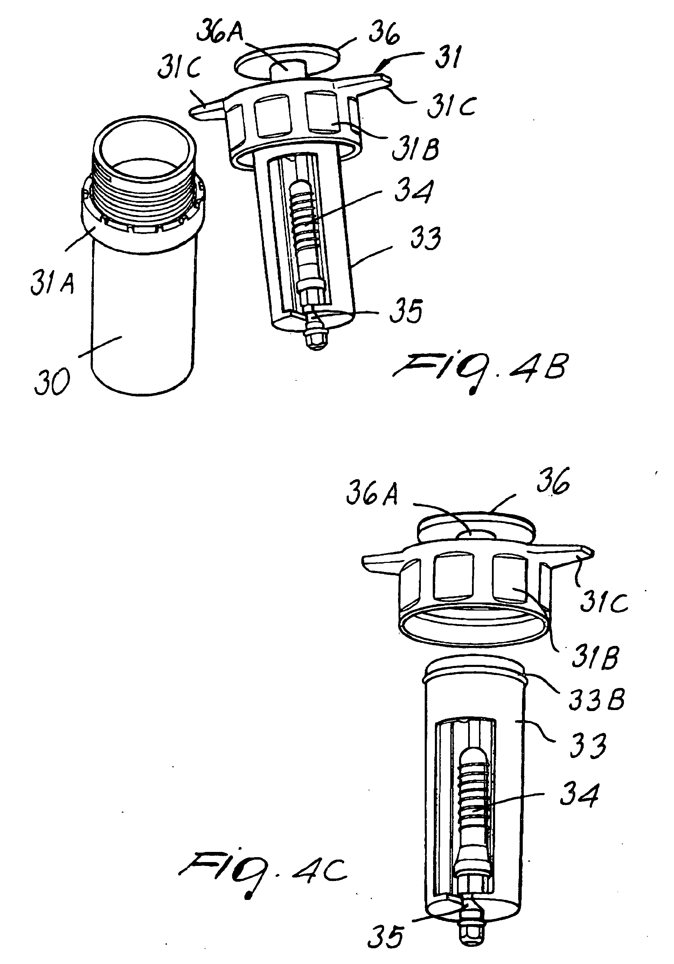

As shown in Figs. 3, 4A and 4B the cap 31 is provided at the lower part thereof with a safety ring 31A which remains in place on the external capsule 30 once the cap 31 is separated from the capsule 30 by breaking the safety ring 31A. The lateral area of the cap 31 is provided with a facetted surface 31B such as to facilitate the gripping thereof by hand or an appropriate tool (not shown). The top surface of the cap 31 is provided with two lateral extensions 31 C which essentially also enable the gripping and the turning of the cap 31.

According to the first embodiment of the present invention a knob 36 is provided at the upper surface of the cap 31, the knob 36 having a lower hollow cylindrical portion 36A which engages an upward projection 31D of the cap 31. The lower portion 31E of the cap 31 extending below the projection 31D is adapted to rest on a top portion 33A of the ampoule 33 in the stored state of the dental implant, as shown in Fig. 3. Furthermore, a downward extension 31F of the cap 31 which protrudes between the external capsule 30 and the ampoule 33 is snap engaged into a groove 33B of the ampoule 33. The complementary solution with a groove on the downward extension 31F of the cap 31 and a corresponding snap protrusion on the ampoule 33 is envisaged as being within the scope of the present invention. Both the downward extension 31F and the groove 33B may have a partial or full circumferential extension as long as a sufficient snap force is provided to remove the ampoule 33 from the external capsule 30 with the ampoule 33 engaged with the cap 31 as shown in Fig. 4B.

According to one aspect of the first embodiment of the present invention the top portion 31G of the cap 31 extending around the projection 31D is made of a resilient or elastic material, such that upon depressing the knob 36 in a downward direction the elastic top portion 31G of the cap 31 moves downwards and the cap 31 may become disengaged from the ampoule 33, as shown in Fig. 4C. In other words, the depression of the knob 36 causes the separation of the downward extension 31F from the groove 33B. As suggested above, the knob 36 may be replaced by lateral wings (not shown) provided in the surface 31B and cooperating with the upper edge of the ampoule 33 such that the axial actuation by depressing the knob to disengage the ampoule is replaced by a lateral actuation by pressing the wings.

The person skilled in the art will appreciate that other alternative solutions for conveying the downward movement of the knob 36 to the top portion 33A of the ampoule 33 may be provided and are encompassed by the scope of the present invention as defined in the appended claims. For instance the knob 36 may be slidingly accommodated in an opening of the cap 31, such that it directly interacts with the top portion 33A of the ampoule 33 causing its separation from the external capsule 30. However, in this alternative, the overall sealing performance of the device, which is highly desirable for the medical tool of the present invention, is diminished.

The operation of the first embodiment of the present invention is as follows. A non-sterile assistance unscrews the cap 31 from external capsule 30 and separates the cap 31 with the ampoule 33 holding the dental implant 34 from the external capsule 30 by gripping with one hand, for instance, the lateral extension 31C and with the other hand the external capsule 30, as shown in Fig. 4B. Furthermore, the cap 31 with the ampoule 33 and the dental implant 34 is brought over the sterile surgery area. Finally, the ampoule 33 with the dental implant 34 is separated from the cap 31 by axially depressing the knob 36 and falls practically into the sterile surgery area. Therefore, any undesired interference of the non-sterile assistance with the dental implant 34 is avoided and the removal of the ampoule 33 with the cap 31 from the external capsule 30 is no longer a problem. The fluid 32, if used, remains within the external capsule 30 and the dental implant 34 is delivered to the dentist or surgeon in the same state as in that of US Patent No. 6 261 097, as apparent from present Fig. 4C.

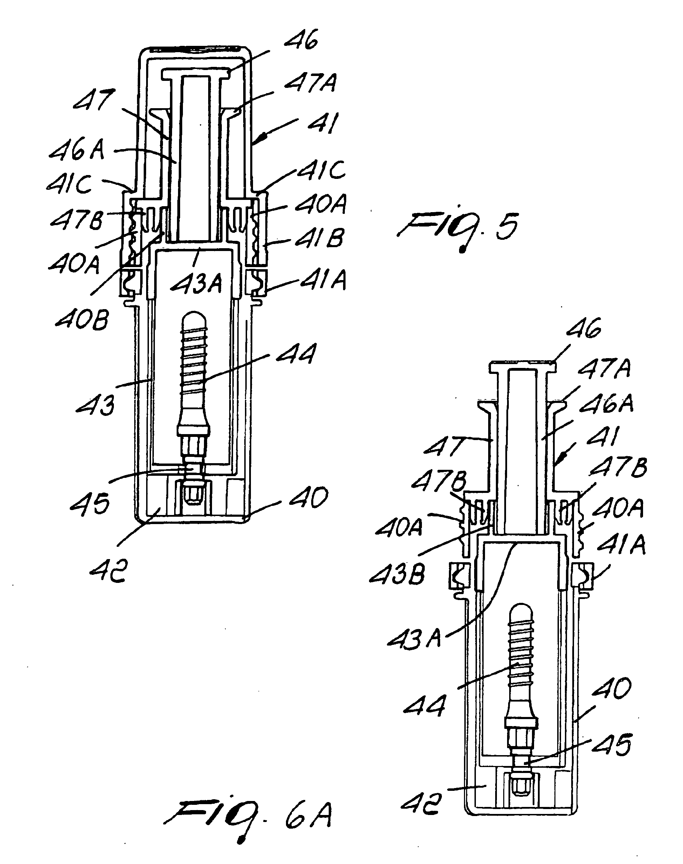

With reference to Figs. 5 and 6A through 6C there is shown a second embodiment of a package with an external capsule for preserving a medical device, in particular a dental implant, according to the present invention. As shown in Fig. 5, the external capsule, which may have a substantially cylindrical shape, is indicated by reference numeral 40 and is closed by a cap 41. The capsule 40 may be made of a transparent material or an opaque material. Transparent materials are preferred.

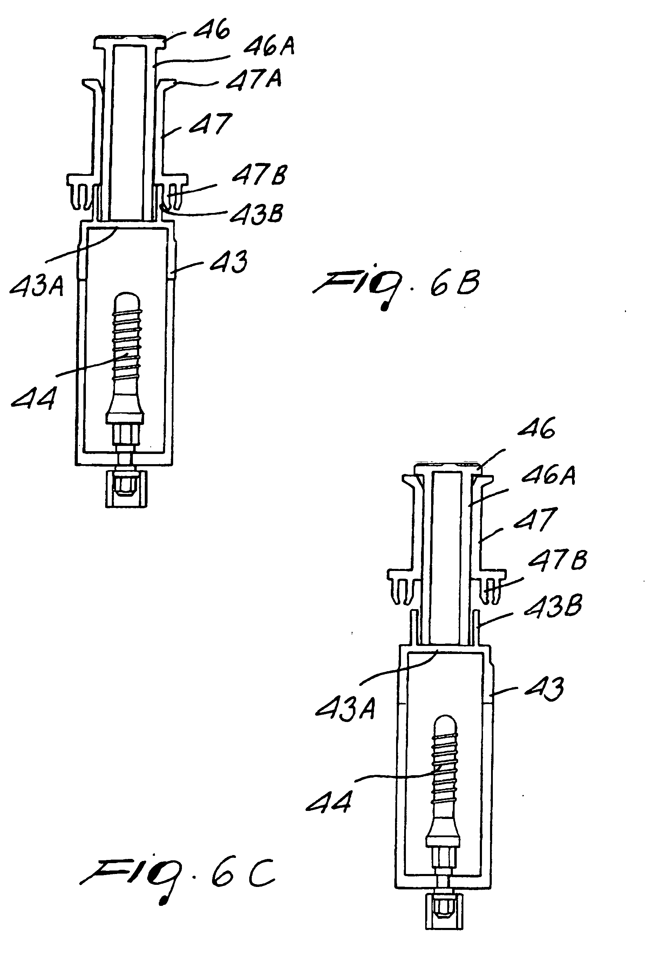

As shown in Figs. 5, 6A and 6B the cap 41 is provided at the lower part thereof with a safety ring 41 A which remains in place on the external capsule 40 once the cap 41 is separated from the capsule 40 by breaking the safety ring 41A. The lateral area 41B of the cap 41 may be adapted to facilitate the gripping thereof by hand or an appropriate tool (not shown).

According to the second embodiment of the present invention the cap 41 clamps a transporter 47 between a shoulder 41 C thereof and the upper edge 40A of the opening of the external capsule 40, such that the inner part of the capsule 40 is provided with an improved seal which is highly desirable and provides advantages for the medical device according to the present invention. As in the case of the first embodiment, the inner part of the capsule 40 may be advantageously filled with a fluid 42, such as an electrolyte or an aqueous solution, and houses an ampoule indicated with reference numeral 43 which holds a dental implant 44 via holding element 45. The ampoule 43, the dental implant 44 and the holding element 45 can be devised and interact in the manner described in the prior art US Patent No 6 261 097 and explained hereinabove. The person skilled in the art will however understand that, in a similar manner to the first embodiment of the invention, the ampoule 43, the dental implant 44 and the holding element 45 may have other alternative configurations without departing from the scope of the present invention as defined in the appended claims.

In the stored state of the dental implant, according to the second embodiment of the invention, the ampoule 43 holding the dental implant 44 is held in place at the upper edge 40A of the opening of the external capsule 40 by a press-fit mechanism designated with reference numeral 47B which engages both a raised peripheral edge 43B of the ampoule 43 and the upper edge 40A of the external capsule 40. A knob 46 is provided with a downwardly extending cylindrical hollow section 46A which is slidingly engaged into the transporter 47. The transporter 47 includes rim 47A that limits the sliding motion of the knob 46 and facilitates gripping. The lower section of the cylindrical hollow section 46A is accommodated in a space defined by a top portion 43A of the ampoule 43 and a raised peripheral edge 43B of the ampoule 43 in such a manner that the lowermost part of the cylindrical hollow section 46A substantially contacts the top portion 43A of the ampoule 43 and a gap is left between the raised peripheral edge 43B of the ampoule 43 the cylindrical hollow section 46A. By virtue of the gap an easy separation of the knob 46 (along with the transporter 47) from the ampule 43 is possible, as described hereinbelow with reference to Fig. 6C.

As shown in Fig. 6A, in a first step, the cap 41 is unscrewed from the external capsule 40 leaving the transporter 47, the knob 46, the ampoule 43 with the dental implant 44 and the capsule 40 in an assembled state.

Furthermore, as shown in Fig. 6B, in a second step, the transporter 47, the knob 46 and the ampoule 43 with the dental implant 44 are removed from the external capsule 40 as one unit, as the press-fit mechanism 47B of the transporter 47 still presses on the raised peripheral edge 43B of the ampoule 43. At this stage the ampoule 43 with the dental implant 44 are removed from the fluid 42 in the external capsule 40, if any. Preferably the removing of the unit formed by the transporter 47, the knob 46 and the ampoule 43 occurs by gripping the transporter 47 at the rim 47A (with one hand) and the external capsule 40 (with the other hand) and pulling in different directions.

Finally, as indicated in Fig. 6C, when the knob 46 is depressed, the press-fit mechanism 47B of the transporter 47 becomes disengaged from the raised peripheral edge 43B of the ampoule 43, thus allowing separation of the ampoule 43 from the transporter 47 and the knob 46.

The operation of the second embodiment of the present invention is as follows. A non-sterile assistance unscrews and separates the cap 41 from external capsule 40. Subsequently, the non-sterile assistance removes the ampoule 43 holding the dental implant 44 from the external capsule 40 (and the fluid contained in the capsule, if any) by gripping with one hand, for instance, the transporter 47 and with the other hand the external capsule 40. Thereafter, the unit comprised of the ampoule 43 holding the dental implant 44, the transporter 47 and the knob 46 is brought over the sterile surgery area. Finally, the ampoule 43 with the dental implant 44 is separated from the transporter 47 and the knob 46 by axially depressing the latter and falls into the sterile surgery area. Therefore, any undesired interference of the non-sterile assistance with the dental implant 44 is avoided and the removal of the ampoule 43 with the transporter 47 from the external capsule 40 is no longer a problem. The fluid 42, if used, remains within the external capsule 40 and the dental implant 44 is delivered to the dentist or surgeon in the same state as in that of US Patent No. 6 261 097, as apparent from present Fig. 6C.

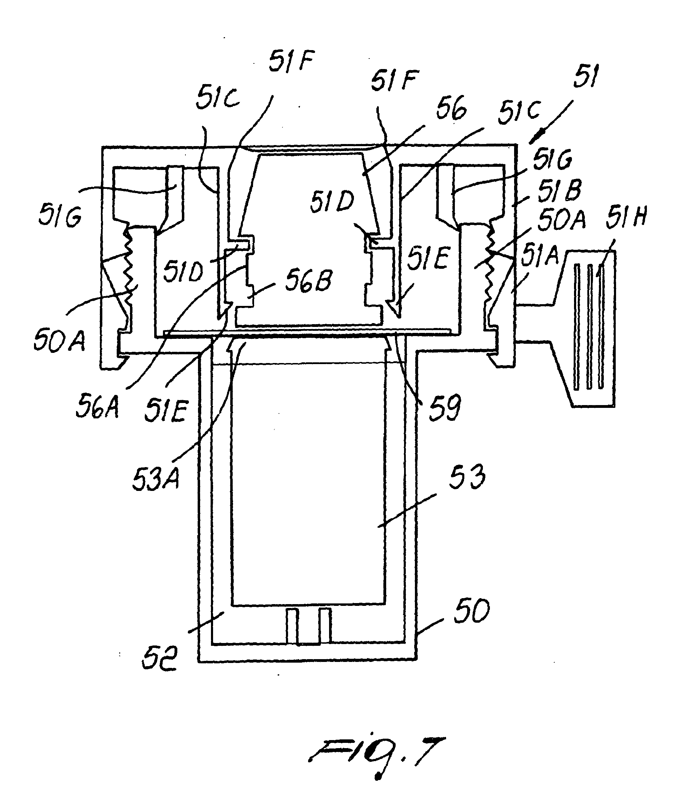

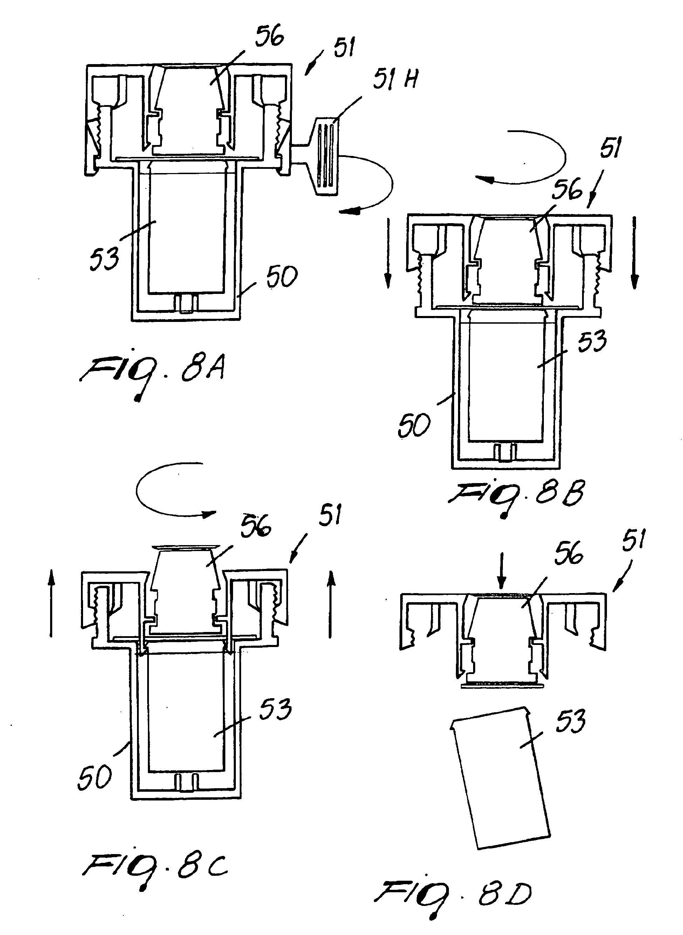

With reference to Figs. 7 and 8A through 8D there is shown a third embodiment of a package with an external capsule for preserving a dental implant or a similar medical device according to the present invention. As shown in Fig. 7, the external capsule, which may have a substantially cylindrical shape, is indicated by reference numeral 50 and is closed by a cap 51. The capsule 50 may be made of a transparent material or an opaque material. Transparent materials are preferred.

Preferably the cap 51 engages the open portion of the external capsule 50 threadingly to provide for a sealed enclosure within the capsule 50. Advantageously the sealed enclosure may be filled with a fluid 52, such as an electrolyte or an aqueous solution, and houses an ampoule indicated with reference numeral 53 which may hold a dental implant (not shown). The seal may be increased by a membrane 59 provided in respect to the top opening of the external capsule 50.

The ampoule 53 can be devised in the manner described in the prior art US Patent No 6 261 097. The person skilled in the art will however appreciate that the ampoule 53 may have other alternative configurations without departing from the scope of the present invention as defined in the appended claims.

As shown in Fig. 7 the cap 51 is provided at the lower part thereof with a tear-off ring 51 A having a gripping tab 51 H extending in a lateral direction. The lateral area of the cap 51 is provided with a suited surface 51B such as to facilitate the gripping thereof by hand or an appropriate tool (not shown).

According to the third embodiment, the cap 51 is provided with a downwardly extending first engagement means 51C which may be embodied as a plurality of discrete arms or a cylindrical section. The first engagement means 51C includes a lateral extension 51D adapted for engaging a first circular groove 56A or a second circular groove 56B which are both provided in a parallel spaced relationship on a knob 56. The cap 51 is also provided at its first engagement means 51C with a snap mechanism 51E adapted for snap coupling with a top portion 53A of the ampoule 53 when the cap 51 is screwed in a downward direction as will be described hereinafter.

The upper surface of the cap 51 may be provided with a predetermined breaking line 51F, the circumference of which corresponds to that of widest cross section of the substantially cylindrical shaped knob 56. Alternatively, the top surface of the knob 56 may be sized, such as to sealingly engage an opening in the cap 51 corresponding to the surface defined by the breaking line 51F.

Preferably, the cap 51 may be provided with a downwardly extending second engagement means 51G which interacts with an upper portion 50A of the capsule 50 when the cap 51 is screwed in a downward direction as will be described hereinafter. Advantageously, the second engagement means 51G provides for a better sealing by the interaction with the upper portion 50A of the capsule 50.

The operation of the third embodiment of the present invention is as follows.

A non-sterile assistance removes in a first step the tear-off ring 51A as indicated in Fig. 8A by gasping the tab 51H. Subsequently, the non-sterile assistance screws the cap 51 down, as shown in Fig. 8B, such that the snap mechanism 51E perforates and cuts the membrane 59 and engages the top portion 53A of the ampoule 53 in the position shown in Fig. 8C. As may be seen in Fig. 8C, the lateral extension 51D of the first engagement means 51 C is now disengaged from first circular groove 56A (to which it was engaged in the state shown in Fig. 7) and is now within the second circular groove 56B of the knob 56. Also during the downward screwing motion of the cap 51 the knob 56 penetrates the breaking line 51F and extends past the upper surface of the cap 51 as seen in Fig. 8C. At this stage the cap 51 is engaged with the knob 56 and the ampoule 53 thus forming a unit. As next, the assistance may unscrew the cap 51 and separate the unit comprised of the cap 51, the knob 56 and the ampoule 53 from the external capsule 50 (and the fluid contained in the capsule, if any). Thereafter, the unit comprised of the ampoule 53, knob 56 and the cap 51 is brought over the sterile surgery area. Finally, the ampoule 53 is separated from the unit in the sterile surgery area by axially depressing the knob 56 as shown in Fig. 8D. Therefore, any undesired interference of the non-sterile assistance with a dental implant is avoided and the removal of the ampoule 53 from the external capsule 50 is no longer a problem.

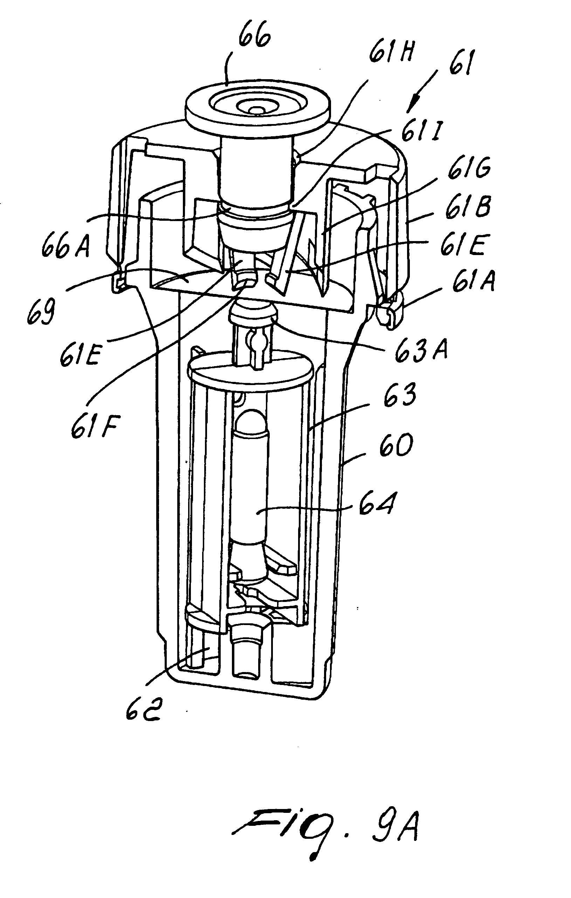

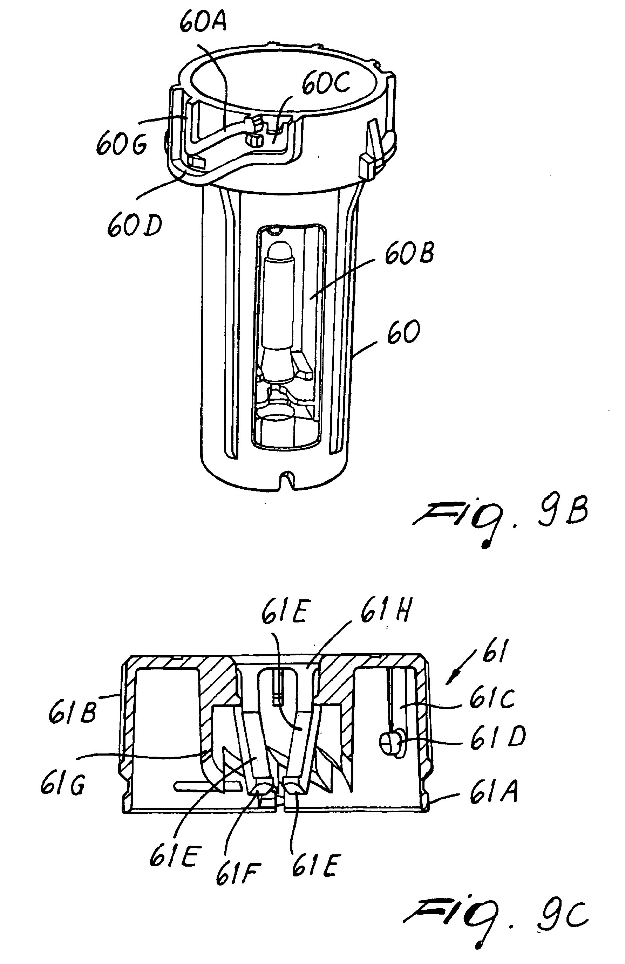

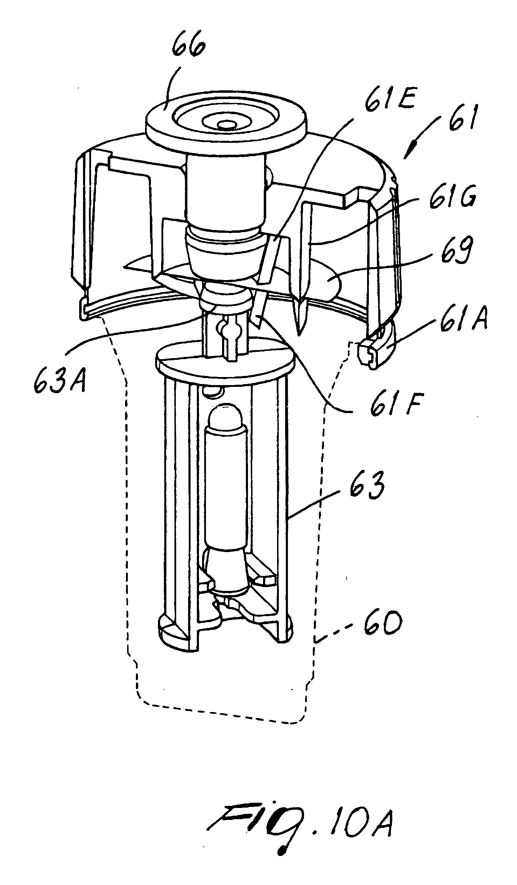

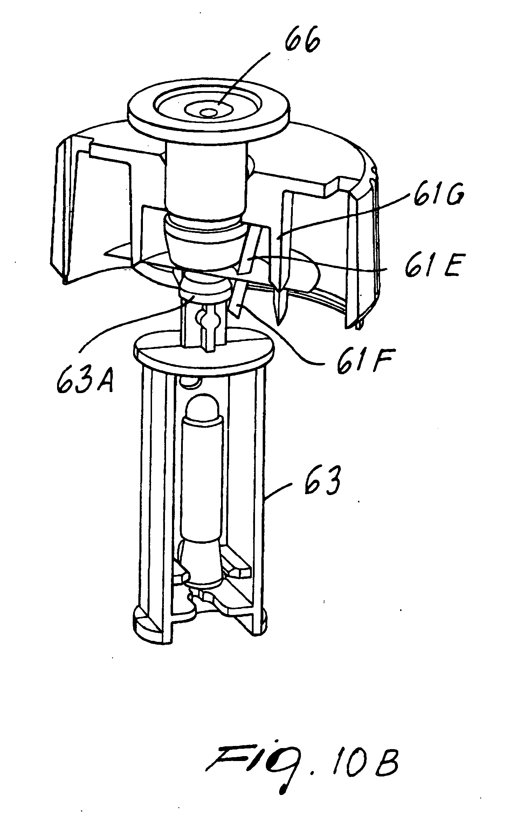

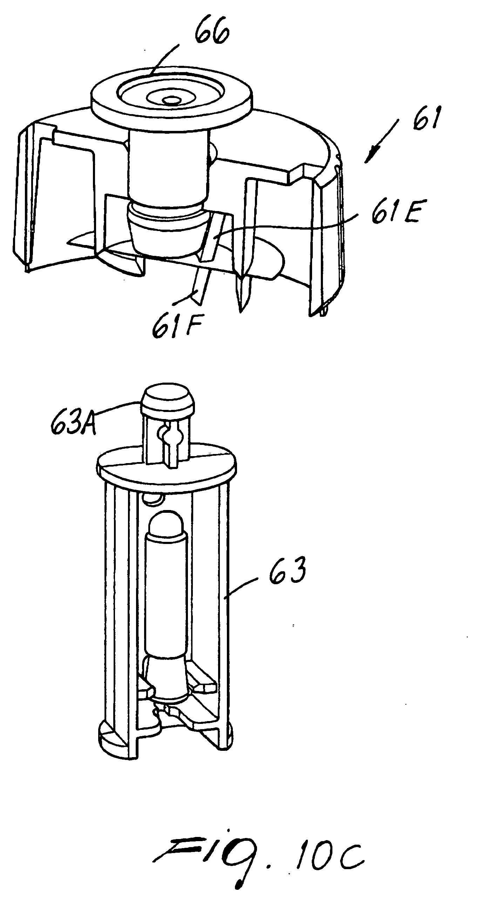

With reference to Figs. 9A through 10C there is shown a fourth embodiment of a package with an external capsule for preserving a dental implant or a similar medical device according to the present invention. As shown in Fig. 10, the external capsule, which may have a substantially cylindrical shape, is indicated by reference numeral 60 and is closed by a cap 61. The capsule 60 may be made of a transparent or an opaque material. Transparent materials are preferred.

Preferably the cap 61 engages the open portion of the external capsule 60 in a bayonet lock 60A. To this end, the cap 61 is provided with downwardly extending arms 61C having lock knobs 61D. Furthermore, the open portion of the external capsule 60 is sealed with a membrane 69 for defining a sealed space or enclosure within the external capsule 60. Advantageously the sealed enclosure may be filled with a fluid 62, such as an electrolyte or an aqueous solution, and houses an ampoule indicated with reference numeral 63 which may hold a dental implant 64 via a holding element (not shown). The ampoule 63 can be devised in the manner described in the prior art US Patent No 6 261 097. The person skilled in the art will however appreciate that the ampoule 63 may have other alternative configurations without departing from the scope of the present invention as defined in the appended claims. The ampoule 63 is provided with a vision field, indicated at 60B, which may be devised in form of an opening therein or as a section made of a transparent material.

As shown in Figs. 9A and 9C the cap 61 is provided at the lower part thereof with a safety ring 61A which is broken upon removal of the cap 61 from the external capsule 60 and remains attached thereto. The lateral area of the cap 61 is provided with a suited surface 61B such as to facilitate the gripping thereof by hand or an appropriate tool (not shown).

According to the fourth embodiment, the cap 61 is provided with a downwardly extending first means 61E which may be embodied as a plurality of discrete arms as shown in Figs. 9A and 9C or as a cylindrical section. The first means 61E includes a lateral extension 61F adapted for engaging a tip end portion 63A of the ampoule 63, as will be explained below. Advantageously, the tip end portion 63A has a knob like shape, such that it can be fully engaged by the lateral extension 61F. Additionally, the first means 61E may also be adapted to perforate and/or cut the membrane 69.

The cap 61 has also a downwardly extending perforating and cutting means 61G which may be embodied as a plurality of discrete arms or as a cylindrical section and which is adapted to perforate and cut the membrane 69, as will be explained hereinafter. Preferably, the perforating and cutting means 6 1 G may be slightly longer than the first means 6 1 E, such that the perforating and cutting action of the perforating and cutting means 61 G occurs prior to the any action of the first means 61E.

A knob 66 is received in an opening 6 1 H of the cap 61 and is held in place by a lock mechanism formed of a groove 66A on the knob 66 with a flange 61I on the cap 61. As will be appreciated by the person skilled in the art, the flange may be provided on the knob and the groove on the cap without departing from the scope of the invention as defined by the appended claims.

The operation of the fourth embodiment according to the present invention is as follows.

With the cap 61 in the upward position, as shown in Fig.9A, a non-sterile assistance moves the cap 61 in a downward direction by rotating the cap 61 in the bayonet lock 60A from the upper position indicated with 60C on the external capsule 60 to the lower position indicated by 60D on the external capsule 60. Although the bayonet lock 60A is shown as having an upright or vertical section at about position 60G, such that extraction of the cap 61 from the external capsule 60 necessitates a rotational and a vertical motion, it has been found that advantageously the bayonet lock can be shaped in a different manner, for instance s-shaped, for allowing the extraction of the cap with a rotational movement only.

As shown in Fig. 10A, when the cap is fully moved in the downward direction, the membrane 69 is perforated and cut by the perforating and cutting means 61G (and possibly by the first means 6 1 E).

Furthermore, the lateral extensions 61F of the first means 61E become engaged with the tip end portion 63A of the ampoule 63, whereupon the cap 61 (with the knob 66 held in place therein) and the ampoule 63 form a unit. In this position the tip end portion (63A) contacts the lower surface of the knob (66).

As next, the assistance may turn the cap 61 and pull in the upward direction from the position 60D in the bayonet lock 60A and completely disengage the unit formed by the cap 61 (with the knob 66) and the ampoule 63 from the external capsule 60 (and the fluid contained in the capsule, if any), as shown in Fig. 10B. In this state the safety ring 61A remains engaged with the external capsule 60.

Thereafter, the unit comprised of the cap 61 and the ampoule 63 is brought over the sterile surgery area. Finally, the ampoule 63 is separated from the unit in the sterile surgery area by axially depressing the knob 66 as shown in Fig. 10C. Therefore, any undesired interference of the non-sterile assistance with a dental implant is avoided and the removal of the ampoule 63 from the external capsule 60 is no longer a problem.

It has been found in respect to all above embodiments and also in respect to conventional external capsules, such as those described in US Patent No. 6 261 097, that the external capsule may be very advantageously manufactured from cyclo-olefin copolymer (COC) or the like, which is a plastic material with an excellent impermeability to moisture (less than 5%, preferably less than 1% fluid loss per year) and good impermeability to gas. At the same time COC is transparent and can be sterilized and has full medical device certification (FDA, CE). COC is also advantageously used to manufacture the ampoule in view of its good hydrophobic properties (less than 0.01% fluid absorption in 24 hours at 23°C), such that the overall shape of the ampoule does not change while immersed in a fluid.

The cap may be advantageously manufactured from a polymer. High density polyethylene (HDPE) or low density polyethylene (LDPE) has proven particularly advantageous for caps. Also polypropylene (PP) has proven advantageous for caps according to the present invention, particularly for those according to the fourth embodiment thereof. Nevertheless, as above, the HDPE, LPDE or PP cap can be applied to all above embodiments and also in respect to conventional external capsules, such as those described in US Patent No. 6 261 097.

Further, the cap can be replaced by a sealing barrier or used together with the barrier, such that a particularly good impermeability is provided in conjunction with the COC external capsule. Preferably, the sealing barrier is embodied as an aluminum membrane. Nevertheless, titanium or polymer membranes can also be used.

In addition, the combination of a COC capsule with a HDPE or LDPE cap and/or the aluminum barrier provides for an excellent shelf life of the medical device stored therein, particularly if a storage fluid, such as an electrolyte or an aqueous solution, is used. The shelf life is further improved by the quality of the seal between the capsule and the cap.

The foregoing description of the invention, including the preferred embodiments thereof, has been presented for the purpose of illustration and description.

A person skilled in the art will readily understand that the external capsule and the ampoule are not limited to the use with dental implants. Rather according to the invention the external capsule and the ampoule may by used in connection with other medical devices providing the same handling and sterility maintenance advantages as described hereinbefore.

The embodiments described are chosen to provide an illustration of principles of the invention and its practical application to enable thereby one of ordinary skill in the art to utilize the invention in various embodiments and with various modifications as are suited to the particular use contemplated. Therefore, the foregoing description is to be considered exemplary, rather than limiting, and the true scope of the invention is that described in the following claims.

Where technical features mentioned in any claim are followed by reference signs, those reference signs have been included just for the sole purpose of increasing intelligibility of the claims and accordingly, such reference signs do not have any limiting effect on the scope of each element identified by way of example by such reference signs.

## Claims

1. A medical device package comprising an external capsule (30; 50; 60), a cap (31; 51; 61) sealingly engaging the external capsule (30; 50; 60), and an ampoule (33; 53; 63) for holding the medical device, **characterized in that**
a means is provided for releasingly connecting the ampoule (33, 53; 63) with the cap (31; 51; 61) upon removing the cap (31; 51; 61) and the ampoule (33; 53; 63) from the external capsule (30; 50; 60), and wherein the means for releasingly connecting the ampoule (33; 53; 632) with the cap (31; 51; 61) comprises an actuation knob (36; 56; 66) extending at an external surface of the cap (31; 51; 61), the actuation knob (36; 56; 66) being adapted to cause separation of the ampoule (33; 53; 63) from the cap (31; 51; 61) when actuated in an axial direction of the external capsule (30; 50; 60).

2. The package according to claim 1, wherein the means for releasingly connecting the ampoule (33; 53; 63) with the cap (31; 51; 61) comprises a snap coupling between the ampoule (33; 53; 63) and the cap (31; 51; 61), the snap coupling being releasable in an axial direction of the external capsule (30; 50; 60).

3. The package according claims 1 or 2, wherein the cap (31) is threadingly engaged with the external capsule (30) and includes a downward extension (31F) which, in an assembled state of the package, protrudes between the external capsule (30) and the ampoule (33) to snap engage into a groove (33B) of the ampoule (33),

4. The package according to claim 3, wherein the cap (31) comprises a top portion (3 1 G) made of a resilient or elastic material which is in contact with the ampoule (33), such that upon axially depressing a knob (36) operatively connected to the top portion (31G) of the cap (31) the downward extension (31F) of the cap (31) becomes disengaged from a circular groove (33B) of the ampoule (33), thereby separating the ampoule (33) from the cap (31).

5. The package according to claims 1 or 2, wherein the cap (51) comprises a first engagement means (51 C) for engaging a knob (56), which is accommodated within the cap (51), and for selectively engaging the ampoule (53) upon a downward movement of the cap (51) in respect to the axis of the external capsule (50), such that the knob (56) and the ampoule (53) are both engaged, and wherein preferably upon the downward movement of the cap (51) the knob (56) protrudes from the cap (51), such that upon depressing a knob (56) the first engagement means (51C) separates from the ampoule (53).

6. The package according to claims 1, 2 or 5, and further comprising a membrane (59) provided in respect to the top opening the external capsule (50).

7. The package according to claim 6, when dependent on claim 5, wherein the membrane (59) is perforated and cut by a snap mechanism (51E) provided on the first engagement means (51C), the snap mechanism (51) perforating and cutting the membrane (59) upon the downward movement of the cap (51), the snap mechanism (51B) further providing for coupling with the ampoule (53) upon the downward movement of the cap (51).

8. A medical device package comprising an external capsule (40), a cap (41) sealingly engaging the external capsule (40), an ampoule (43) for holding the dental implant, and a transport means surrounded by the cap (41) and extending therein,
wherein the transport means is adapted for releasingly connecting to the ampoule (43), and
wherein the transport means slidingly accommodates a knob (46) extending at an external surface of the cap (41), wherein, in the closed state of the package, the knob (46) contacts the ampoule (43), the knob (46) being adapted to cause separation of the ampoule (43) from the transport means when actuated in an axial direction of the external capsule (40).

9. The package according to claim 8, wherein the transport means comprises a press-fit mechanism (47B) which releasably engages both the ampoule (43) and the external capsule (40).

10. The package according to claim 8, wherein the press-fit mechanism (47B) becomes separated from the ampoule (43) when the knob (46) is actuated in a downward axial direction of the external capsule (40).

11. The package according to any of claims 8 to 10, wherein the transport means defines a seal between the opening of the external capsule (40) and the cap (41) in the assembled state of the device.

12. The package according to claims 1 or 2, further comprising a sealing means (69) closing an open end of the capsule (60).

13. The package according to claim 12, wherein the cap (61) engages the external capsule (60) by means of a bayonet lock (60A), and wherein the sealing means (69) comprises a membrane.

14. The package according to claims 12 or 13, wherein the knob (66) is slidingly received in an opening (61H) of the cap (61) and is held in place by a lock mechanism formed of a groove (66A) on the knob (66) with a flange (61I) on the cap (61), when the package is in the closed state.

15. The package according to any of claims 11-14, wherein the cap (61) comprises a first means (61E) for engaging the ampoule (63) upon a downward movement of the cap (61) in respect to the axis of the external capsule (60), such that the knob (66), which is snappingly held in place in the cap (61), and the ampoule (63) are mutually engaged, and wherein the knob (66) protrudes from the cap (61), such that upon depressing a knob (66) the first means (61E) separates from the ampoule (63).

16. The package according to claim 15, wherein the ampoule (63) has a tip end portion (63A) which engages the first means (61E) upon the completion of the downward movement of the cap (61), such that the tip end portion (63A) contacts the lower surface knob (66).

17. The package according to claims 15 or 16, wherein the cap (61) further comprises a perforating and cutting means (61G) for perforating and cutting the sealing means (69) upon the downward movement of the cap (61) in respect to the axis of the external capsule (60), and wherein preferably the perforating and cutting means (61G) is slightly longer than the first means (61E), such that during the downward movement the perforating and cutting action thereof occurs prior to the engagement of the first means (61E).

18. The package according to any of the preceding claims, further comprising a fluid (32; 42; 52; 62), such as an electrolyte or an aqueous solution, within the external capsule (30; 40; 50; 60).

19. The package according to any of the preceding claims, wherein the external capsule (30; 40; 50; 60) is made of cyclo-olefin copolymer, and/or wherein the ampoule (33; 43; 53; 63) is made of cyclo-olefin copolymer, and/or wherein the cap (31; 41; 51; 61) is made of high density polyethylene or low density polyethylene.

20. The package according to any of the preceding claims, wherein the package is a dental implant package.

## Patentansprüche

1. Ein medizinisches Vorrichtungs-Paket, das Folgendes umfasst: eine äußere Kapsel (30; 50; 60), eine Kappe (31; 51; 61), die die äußere Kapsel (30; 50; 60) abdichtend hält, und eine Ampulle (33; 53; 63) zum Halten der medizinischen Vorrichtung, **dadurch gekennzeichnet, dass**
ein Mittel bereitgestellt wird, um die Ampulle (33; 53; 63) beim Entfernen der Kappe (31; 51; 61) und der Ampulle (33, 53; 63) aus der äußeren Kapsel (30; 50; 60) lösbar zu verbinden, und wobei das Mittel zur lösbaren Verbindung der Ampulle (33; 53; 63) mit der Kappe (31; 51; 61) einen Betätigungsknopf (36; 56; 66) umfasst, der sich an einer äußeren Oberfläche der Kappe (31; 51; 61) erstreckt, wobei der Betätigungsknopf (36; 56; 66) ausgebildet ist, um die Trennung der Ampulle (33; 53; 63) von der Kappe (31; 51; 61) zu veranlassen, wenn er in einer axialen Richtung der äußeren Kapsel (30; 50; 60) betätigt wird.

2. Das Paket gemäß Anspruch 1, wobei das Mittel zur lösbaren Verbindung der Ampulle (33; 53; 63) mit der Kappe (31; 51; 61) eine Schnappverbindung zwischen der Ampulle (33; 53; 63) und der Kappe (31; 51; 61) umfasst und die Schnappverbindung in einer axialen Richtung der äußeren Kapsel (30; 50; 60) lösbar ist.

3. Das Paket gemäß Anspruch 1 oder 2, wobei die Kappe (31) mit dem Gewinde in die äußere Kapsel (30) eingreift und eine Abwärtsverlängerung (31F) einschließt, welche in einem zusammengebauten Zustand des Pakets zwischen der äußeren Kapsel (30) und der Ampulle (33) herausragt, um schnappend in eine Rille (33B) der Ampulle (33) einzugreifen.

4. Das Paket gemäß Anspruch 3, wobei die Kappe (31) einen oberen Abschnitt (31G) aus federndem oder elastischem Material umfasst, der in Kontakt mit der Ampulle (33) steht, so dass beim axialen Herunterdrücken eines Knopfs (36), der operativ mit dem oberen Abschnitt (31 G) der Kappe (31) verbunden ist, die Abwärtsverlängerung (31 F) der Kappe (31) aus einer Ringnut (33B) der Ampulle (33) gelöst wird, wodurch die Ampulle (33) von der Kappe (31) getrennt wird.

5. Das Paket gemäß Anspruch 1 oder 2, wobei die Kappe (51) ein erstes Eingriffsmittel (51C) zum Eingreifen eines Knopfs (56) umfasst, der in der Kappe (51) untergebracht ist, und zum selektiven Eingreifen der Ampulle (53) bei einer Abwärtsbewegung der Kappe (51) mit Bezug auf die Achse der äußeren Kapsel (50), so dass sowohl der Knopf (56) als auch die Ampulle (53) eingegriffen werden, und wobei vorzugsweise bei Abwärtsbewegung der Kappe (51) der Knopf (56) aus der Kappe (51) herausragt, so dass beim Herunterdrücken eines Knopfs (56) das erste Eingriffsmittel (51C) von der Ampulle (53) getrennt wird.

6. Das Paket gemäß Anspruch 1, 2 oder 5, das weiter eine Membran (59) umfasst, die mit Bezug auf die obere Öffnung der äußeren Kapsel (50) bereitgestellt ist.

7. Das Paket gemäß Anspruch 6, wenn abhängig von Anspruch 5, wobei die Membran (59) von einem Schnappmechanismus (51E) perforiert und geschnitten wird, der auf den ersten Eingriffsmitteln (51C) bereitgestellt ist, wobei der Schnappmechanismus (51E) die Membran (59) bei Abwärtsbewegung der Kappe (51) perforiert und schneidet und der Schnappmechanismus (51E) weiter für die Kopplung mit der Ampulle (53) bei Abwärtsbewegung der Kappe (51) sorgt.

8. Ein medizinisches Vorrichtungs-Paket, das Folgendes umfasst: eine äußere Kapsel (40), eine Kappe (41), welche die äußere Kapsel (40) abdichtend hält, eine Ampulle (43) zum Halten des Zahnimplantats und ein Beförderungsmittel, das von der Kappe (41) umgeben ist und sich darin erstreckt,
wobei das Beförderungsmittel zur lösbaren Verbindung mit der Ampulle (43) ausgebildet ist und
wobei das Beförderungsmittel verschiebbar einen Knopf (46) aufnimmt, der sich an einer äußeren Oberfläche der Kappe (41) erstreckt, wobei im geschlossenen Zustand des Pakets der Knopf (46) die Ampulle (43) berührt und der Knopf (46) ausgebildet ist, um die Trennung der Ampulle (43) vom Beförderungsmittel zu veranlassen, wenn er in einer axialen Richtung der äußeren Kapsel (40) betätigt wird.

9. Das Paket gemäß Anspruch 8, wobei das Beförderungsmittel einen Presspassungsmechanismus (47B) umfasst, der sowohl die Ampulle (43) als auch die äußere Kapsel (40) lösbar hält.

10. Das Paket gemäß Anspruch 8, wobei der Presspassungsmechanismus (47B) von der Ampulle (43) getrennt wird, wenn der Knopf (46) in einer axialen Abwärtsrichtung der äußeren Kapsel (40) betätigt wird.

11. Das Paket gemäß einem beliebigen der Ansprüche 8 bis 10, wobei das Beförderungsmittel im zusammengebauten Zustand der Vorrichtung eine Abdichtung zwischen der Öffnung der äußeren Kapsel (40) und der Kappe (41) bestimmt.

12. Das Paket gemäß Anspruch 1 oder 2, das weiter ein Abdichtungsmittel (69) umfasst, das ein offenes Ende der Kapsel (60) verschließt.

13. Das Paket gemäß Anspruch 12, wobei die Kappe (61) die äußere Kapsel (60) mit Hilfe eines Bajonettverschlusses (60A) hält und wobei das Abdichtungsmittel (69) eine Membran umfasst.

14. Das Paket gemäß Anspruch 12 oder 13, wobei der Knopf (66) verschiebbar in einer Öffnung (61H) der Kappe (61) aufgenommen ist und an Ort und Stelle gehalten wird durch einen Verriegelungsmechanismus, bestehend aus einer Rille (66A) am Knopf (66) mit einem Flansch (61I) an der Kappe (61), wenn das Paket sich im geschlossenen Zustand befindet.

15. Das Paket gemäß einem beliebigen der Ansprüche 11-14, wobei die Kappe (61) ein erstes Mittel (61E) zum Halten der Ampulle (63) bei einer Abwärtsbewegung der Kappe (61) mit Bezug auf die Achse der äußeren Kapsel (60) umfasst, so dass der Knopf (66), der in der Kappe (61) schnappend an Ort und Stelle gehalten wird, und die Ampulle (63) ineinander eingreifen, und wobei der Knopf (66) aus der Kappe (61) herausragt, so dass beim Herunterdrücken eines Knopfs (66) das erste Mittel (61 E) von der Ampulle (63) getrennt wird.

16. Das Paket gemäß Anspruch 15, wobei die Ampulle (63) einen Spitzenendabschnitt (63A) hat, der nach Beendigung der Abwärtsbewegung der Kappe (61) in das erste Mittel (61E) eingreift, so dass der Spitzenendabschnitt (63A) den unteren Oberflächenknopf (66) berührt.

17. Das Paket gemäß Anspruch 15 oder 16, wobei die Kappe (61) weiter ein Perforations- und Schneidemittel (61G) zum Perforieren und Schneiden der Abdichtungsmittel (69) bei Abwärtsbewegung der Kappe (61) im Verhältnis zur Achse der äußeren Kapsel (60) umfasst und wobei das Perforations- und Schneidemittel (61G) etwas länger ist als das erste Mittel (61E), so dass während der Abwärtsbewegung die Perforations- und Schneidearbeit davon vor dem Eingriff des ersten Mittels (61E) stattfindet.

18. Das Paket gemäß einem beliebigen der obigen Ansprüche, das weiter ein Fluid (32; 42; 52; 62), wie zum Beispiel einen Elektrolyten oder eine wässerige Lösung, in der äußeren Kapsel (30; 40; 50; 60) umfasst.

19. Das Paket gemäß einem beliebigen der obigen Ansprüche, wobei die äußere Kapsel (30; 40; 50; 60) aus Cycloolefincopolymer besteht und/oder wobei die Ampulle (33; 43; 53; 63) aus Cycloolefincopolymer besteht und/oder wobei die Kappe (31; 41; 51; 61) aus Polyethylen mit hoher Dichte oder Polyethylen mit niedriger Dichte besteht.

20. Das Paket gemäß einem beliebigen der obigen Ansprüche, wobei das Paket ein Zahnimplantat-Paket ist.

## Revendications

1. Emballage pour dispositif médical, comprenant une capsule externe (30 ; 50 ; 60), un bouchon (31 ; 51 ; 61) venant en prise, avec effet de fermeture étanche, avec la capsule externe (30 ; 50 ; 60), et une ampoule (33 ; 53 ; 63) pour maintenir le dispositif médical, **caractérisé en ce que**
des moyens sont prévus, pour relier de manière désolidarisable l'ampoule (33 ; 53 ; 63) au bouchon (31 ; 51 ; 61), lors de l'enlèvement du bouchon (31 ; 51 ; 61) et de l'ampoule (33 ; 53 ; 63) vis-à-vis de la capsule externe (30 ; 50 ; 60), et dans lequel les moyens pour relier de manière désolidarisable l'ampoule (33 ; 53 ; 63) au bouchon (31 ; 51 ; 61) comprennent un bouton d'actionnement (36 ; 56 ; 66), s'étendant à une surface externe du bouchon (31 ; 51 ; 61), le bouton d'actionnement (36 ; 56 ; 66) étant adapté pour provoquer la séparation de l'ampoule (33 ; 53 ; 63) vis-à-vis du bouchon (31 ; 51 ; 61) lorsqu'il et actionné dans une direction axiale de la capsule externe (30 ; 50 ; 60).

2. Emballage selon la revendication 1, dans lequel les moyens pour relier de manière désolidarisable l'ampoule (33 ; 53 ; 63) au bouchon (31 ; 51 ; 61) comprennent un accouplement à encliquetage entre l'ampoule (33 ; 53 ; 63) et le bouchon (31 ; 51 ; 61), l'accouplement à encliquetage étant susceptible d'être libéré dans une direction axiale de la capsule externe (30 ; 50 ; 60).

3. Emballage selon la revendication 1 ou 2, dans lequel le bouchon (31) est mis en prise, par filetage, avec la capsule externe (30) et comprend une extension orientée vers le bas (31F) qui, lorsque l'emballage se trouve en un état assemblé, fait saillie entre la capsule externe (30) et l'ampoule (33), pour venir en prise, avec encliquetage, dans une gorge (33B) de l'ampoule (33).

4. Emballage selon la revendication 3, dans lequel le bouchon (31) comprend une partie supérieure (31G), faite d'un matériau résilient ou élastique, placé en contact avec l'ampoule (33), de manière que, lorsqu'un bouton (36), relié fonctionnellement à la partie supérieure (31 G) du bouchon (31), est enfoncé axialement, l'extension orientée vers le bas (31F) du bouchon (31) est dégagée d'une gorge (33B) circulaire de l'ampoule (33), de manière à séparer l'ampoule (33) du bouchon (31).

5. Emballage selon la revendication 1 ou 2, dans lequel le bouchon (51) comprend des premiers moyens de mise en prise (51C), pour venir en prise avec un bouton (56), logé à l'intérieur du bouchon (51), et pour venir en prise, sélectivement, avec l'ampoule (53), lors d'un déplacement, orienté vers le bas, du bouchon (51) par rapport à l'axe de la capsule externe (50), de manière que le bouton (56) et l'ampoule (53) soient tous deux en prise, et dans lequel, de préférence lors du déplacement, orienté vers le bas, du bouchon (51), le bouton (56) fasse saillie du bouchon (51), de manière que, lors de l'enfoncement d'un bouton (56), les premiers moyens de mise en prise (51 C) se séparent de l'ampoule (53).

6. Emballage selon les revendications 1, 2 ou 5, et comprenant en outre une membrane (59) disposée par rapport à l'ouverture supérieure de la capsule externe (50).

7. Emballage selon la revendication 6, dépendant de la revendication 5, dans lequel la membrane (59) est perforée et coupée par un mécanisme à détente brusque (51E), prévu sur les premiers moyens de mise en prise (51C), le mécanisme à détente brusque (51E) perforant et coupant la membrane (59) lors du déplacement, orienté vers le bas, du bouchon (51), le mécanisme à détente brusque (51E) assurant en outre un accouplement à l'ampoule (53) lors du déplacement, orienté vers le bas, du bouchon (51).

8. Emballage pour dispositif médical, comprenant une capsule externe (40), un bouchon (41) venant en prise, avec effet de fermeture étanche, avec la capsule externe (40), une ampoule (43) pour maintenir l'implant dentaire, et des moyens de transport, entourés par le bouchon (41) et s'étendant en son sein,
dans lequel les moyens de transport sont adaptés pour une connexion désolidarisable à l'ampoule (43), et
dans lequel les moyens de transport reçoivent à coulissement un bouton (46) s'étendant à une surface externe du bouchon (41), dans lequel, lorsque l'emballage se trouve à l'état fermé, le bouton (46) entre en contact avec l'ampoule (43), le bouton (46) étant adapté pour provoquer la séparation de l'ampoule (43) vis-à-vis des moyens de transport lorsqu'i lest actionné dans une direction axiale de la capsule externe (40).

9. Emballage selon la revendication 8, dans lequel les moyens de transport comprennent un mécanisme à ajustage serré (47B), mettant en prise, de manière désolidarisable, à la fois l'ampoule (43) et la capsule externe (40).

10. Emballage selon la revendication 8, dans lequel le mécanisme à ajustage serré (47B) se sépare de l'ampoule (43) lorsque le bouton (46) est actionné dans une direction axiale, orientée vers le bas, de la capsule externe (40).

11. Emballage selon l'une quelconque des revendications 8 à 10, dans lequel les moyens de transport définissent une fermeture étanché entre l'ouverture de la capsule externe (40) et le bouchon (41) lorsque le dispositif se trouve à l'état assemblé.

12. Emballage selon la revendication 1 ou 2, comprenant en outre des moyens de fermeture étanche (69), fermant une extrémité ouverte de la capsule (60).

13. Emballage selon la revendication 12, dans lequel le bouchon (61) vient en prise avec la capsule externe (60) au moyen d'un verrouillage à baïonnette (60A), et dans lequel les moyens de fermeture étanche (69) comprennent une membrane.

14. Emballage selon la revendication 12 ou 13, dans lequel le bouton (66) est logé à coulissement dans une ouverture (61H) du bouchon (61) et est maintenu en place par un mécanisme de verrouillage formé d'une gorge (66A) sur le bouton (66), avec une bride (61I) sur le bouchon (61), lorsque l'emballage se trouve à l'état fermé.

15. Emballage selon l'une quelconque des revendications 11 à 14, dans lequel le bouchon (61) comprend des premiers moyens (61E), pour venir en prise avec l'ampoule (63) lors d'un déplacement, orienté vers le bas, du bouchon (61) par rapport à l'axe de la capsule externe (60), de manière que le bouton (66), maintenu en place par encliquetage dans le bouchon (61), et l'ampoule (63) soient mis mutuellement en prise, et dans lequel le bouton (66) fait saillie du bouchon (61), de manière que, lorsqu'un bouton (66) est enfoncé, les premiers moyens (61B) se séparent de l'ampoule (63).

16. Emballage selon la revendication 15, dans lequel l'ampoule (63) comprend une partie d'extrémité de pointe (63A), venant en prise avec les premiers moyens (61E) lors de l'achèvement du déplacement, orienté vers le bas, du bouchon (61), de manière que la partie d'extrémité de pointe (63A) entre en contact avec la surface inférieure du bouton (66).

17. Emballage selon la revendication 15 ou 16, dans lequel le bouchon (61) comprend en outre des moyens de perforation et de découpage (61 G), pour perforer et découper les moyens de fermeture étanche (69) lors du déplacement, orienté vers le bas, du bouchon (61) par rapport à l'axe de la capsule externe (60), et dans lequel, de préférence, les moyens de perforation et de découpage (61 G) sont légèrement plus longs que les premiers moyens (61E), de manière que, durant le déplacement, orienté vers le bas, l'action de perforation et de découpage de ceux-ci s'accomplisse avant l'engagement des premiers moyens (61E).

18. Emballage selon l'une quelconque des revendications précédentes, comprenant en outre un fluide (32 ; 42 ; 52 ; 62), tel qu'un électrolyte ou une solution aqueuse, disposé à l'intérieur de la capsule externe (30 ; 40 ; 50 ; 60).

19. Emballage selon l'une quelconque des revendications précédentes, dans lequel la capsule externe (30 ; 40 ; 50 ; 60) est composée d'un copolymère de cyclo-oléfine, et/ou dans lequel l'ampoule (33 ; 43 ; 53 ; 63) est composée d'un copolymère de cyclo-oléfine, et/ou dans lequel le bouchon (31 ; 41 ; 51 ; 61) est composé de polyéthylène haute densité ou de polyéthylène basse densité.

20. Emballage selon l'une quelconque des revendications précédentes, dans lequel l'emballage est un emballage pour implant dentaire.
